(19) Europäisches Patentamt
European Patent Office
Office européen des brevets

(11) **EP 4 564 045 A1**

(12) **EUROPEAN PATENT APPLICATION**

(43) Date of publication:
04.06.2025 Bulletin 2025/23

(21) Application number: 24214533.2

(22) Date of filing: 21.11.2024

(51) International Patent Classification (IPC):
$G01S\ 7/288^{(2006.01)}$     $A61B\ 5/0507^{(2021.01)}$
$A61B\ 5/08^{(2006.01)}$     $A61B\ 5/00^{(2006.01)}$
$G01S\ 7/292^{(2006.01)}$     $G01S\ 7/41^{(2006.01)}$
$G01S\ 13/02^{(2006.01)}$     $G01S\ 13/10^{(2006.01)}$
$G01S\ 13/88^{(2006.01)}$

(52) Cooperative Patent Classification (CPC):
G01S 13/0209; A61B 5/0507; A61B 5/0816;
A61B 5/7207; G01S 7/2886; G01S 7/2923;
G01S 7/414; G01S 7/415; G01S 13/10; G01S 13/88

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 30.11.2023 US 202363604658 P

(71) Applicant: QORVO US, INC.
Greensboro, NC 27409 (US)

(72) Inventor: MEILHAC, Lisa
Valbonne (FR)

(74) Representative: D Young & Co LLP
3 Noble Street
London EC2V 7BQ (GB)

(54) **METHODS, SYSTEMS, AND DEVICES FOR BREATHING MONITORING**

(57) Embodiments of the present disclosure include methods, systems, and devices for breathing detection and monitoring comprising: receiving, via a data interface, a first parameter; receiving, via a receiver, a plurality of signals; generating a channel impulse response from the plurality of signals; selecting a portion of the channel impulse response based on the first parameter; generating a modified signal from the portion of the channel impulse response, wherein the modified signal is the portion of the channel impulse response with clutter removed; generating a plurality of regression lines from the modified signal; computing a plurality of times based on the plurality of regression lines, wherein each of the plurality of regression lines goes through zero at one of the plurality of times; estimating a zero-crossing time based on the plurality of times; and generating a breathing rate estimation based on the zero-crossing time.

FIG. 1

**Description**

## CROSS REFERENCED APPLICATIONS

This application claims priority to and benefit of U.S. Provisional Applications No. 63/604,658 filed November 30, 2023 and entitled "Methods, Systems, and Devices for Breathing Monitoring," which is incorporated by reference herein in its entirety.

## FIELD OF THE DISCLOSURE

The present disclosure relates to radar devices, systems, and methods for breathing detection and monitoring.

## BACKGROUND

Radar systems, including ultra-wideband-based radars, can be used to sense the environment by providing a means of obtaining propagation channel measures. The propagation channel is due to the reflections of the transmitted signal on the environment. The channel measures usually take the form of a set of periodic channel impulse response estimations (CIRE). Each CIRE's complex components (taps) corresponds to a propagation delay of the reflected signal and thus to a reflecting target's distance.

Small movements of a reflecting target such as those resulting from breathing introduce variations over time of the CIRE tap corresponding to the breathing target distance. As the breathing is usually an approximately periodic operation, this latter CIRE tap will vary according to the same pattern. Thus, breathing detection monitoring algorithm classically consists in applying any of a number of frequency analysis methods (short time Fourier transform, wavelet, MUSIC, ...) to all the CIRE taps to detect the presence of a periodic variation in some of them. This periodic variation would indicate the presence of a breathing person and providing a breathing rate estimation.

However, frequency analysis methods are not robust to movement of subjects, to irregular breathing patterns, or to fast breathing rate variation. Furthermore, they have high complexity due to processing demands.

Thus, improved systems and methods for breathing monitoring and detection are required.

## SUMMARY

Particular aspects are set out in the appended independent claims. Various optional embodiments are set out in the dependent claims. In an example aspect, the present disclosure is directed to a method for breathing detection and monitoring. The method also includes receiving, via a data interface, a first parameter; receiving, via a receiver, a plurality of signals; generating a channel impulse response from the plurality of signals; selecting a portion of the channel impulse response based on the first parameter; generating a modified signal from the portion of the channel impulse response, where the modified signal is the portion of the channel impulse response with clutter removed; generating a plurality of regression lines from the modified signal; computing a plurality of times based on the plurality of regression lines, where each of the plurality of regression lines goes through zero at one of the plurality of times; estimating a zero-crossing time based on the plurality of times; or generating a breathing rate estimation based on the zero-crossing time.

In some aspects, implementations may include one or more of the following features. The method may include sorting the zero-crossing time based on a direction of change of the modified signal at the zero-crossing time. The cost matrix may be based on a difference between the estimated zero-crossing time and a zero-crossing time stored in a track. The method may include assigning the estimated zero-crossing time to the track if a gating constraint is satisfied. The channel impulse response is a channel impulse response estimate. The breathing rate estimation is generated at each of a plurality of time steps. The method may include detecting a false zero-crossing time detection based on one or more confidence criteria. The clutter may include background echoes in an environment.

In an example aspect, the present disclosure is directed to a device. The device also includes a receiver; a memory storing instructions; and one or more processors configured to execute the instructions to cause the device to perform operations that may include: receiving, via a data interface, a first parameter; receiving, via a receiver, a plurality of signals; generating a channel impulse response from the plurality of signals; selecting a portion of the channel impulse response based on the first parameter; generating a modified signal from the portion of the channel impulse response, where the modified signal is the portion of the channel impulse response with clutter removed; generating a plurality of regression lines from the modified signal; computing a plurality of times based on the plurality of regression lines, where each of the plurality of regression lines goes through zero at one of the plurality of times; estimating a zero-crossing time based on the plurality of times; and generating a breathing rate estimation based on the zero-crossing time.

In some aspects, implementations may include one or more of the following features. The device where one or more processors are further configured to execute instructions that may include: sorting the zero-crossing time based on a direction of change of the modified signal at the zero-crossing time. One or more processors are further configured to execute instructions that may include: tracking estimated zero-crossing times based on a cost matrix, where the cost matrix is based on a difference between the estimated zero-crossing time and a zero-crossing time stored in a track; and assigning the estimated zero-crossing time to the track if a gating constraint is satisfied. The channel impulse response is a channel impulse response estimate. The breathing rate estimation is generated at each of a plurality of time steps. One or more processors are further configured to execute instructions that may include: detecting a false zero-crossing time detection based on one or more confidence criteria. The clutter that may include background echoes in an environment.

In an example aspect, the present disclosure is directed to a computer-readable medium encoding instructions which, when executed by one or more processors, cause the one or more processors to: receive, via a data interface, a first parameter; generate a channel impulse response from a plurality of signals; select a portion of the channel impulse response based on the first parameter; generate a modified signal from the portion of the channel impulse response, where the modified signal is the portion of the channel impulse response with clutter removed; generate a plurality of regression lines from the modified signal; compute a plurality of times based on the plurality of regression lines, where each of the plurality of regression lines goes through zero at one of the plurality of times; estimate a zero-crossing time based on the plurality of times; or generate a breathing rate estimation based on the zero-crossing time.

In some aspects, implementations may include one or more of the following features. The computer-readable medium where the instructions, when executed by the one or more processors, further cause the one or more processors to: sort the zero-crossing time based on a direction of change of the modified signal at the zero-crossing time. The instructions, when executed by the one or more processors, further cause the one or more processors to: track estimated zero-crossing times based on a cost matrix, where the cost matrix is based on a difference between the estimated zero-crossing time and a zero-crossing time stored in a track; and assign the estimated zero-crossing time to the track if a gating constraint is satisfied. The channel impulse response is a channel impulse response estimate. The breathing rate estimation is generated at each of a plurality of time steps.

Those skilled in the art will appreciate the scope of the present disclosure and realize additional aspects thereof after reading the following detailed description of the preferred embodiments in association with the accompanying drawing figures.

## BRIEF DESCRIPTION OF THE DRAWING FIGURES

The accompanying drawing figures incorporated in and forming a part of this specification illustrate several aspects of the disclosure, and together with the description, serve to explain the principles of the disclosure.

FIG. 1 illustrates a monitoring scenario using radar.
FIGS. 2A and 2B depict graphs of channel impulse responses.
FIG. 3 depicts a graph of an example periodic signal.
FIG. 4 depicts a graph of a portion of a breathing signal with noise.
FIG. 5 illustrates a block diagram of a breath monitoring system, according to some aspects of the present disclosure.
FIG. 6 illustrates a block flow diagram for detecting zero-crossing events, according to some aspects of the present disclosure.
FIG. 7 illustrates a block diagram for tracking zero-crossing events, according to some aspects of the present disclosure.
FIG. 8 is a simplified diagram of a radar-enabled device, according to some aspects of the present disclosure.
FIG. 9 illustrates an example method for breathing rate estimation and tracking by a radar-enabled device, according to some aspects of the present disclosure.
FIG. 10 illustrates a plot of breathing rate over time for a stationary subject, according to some aspects of the present disclosure.
FIG. 11 illustrates a plot of breathing rate over time for a stationary subject using a frequency-based technique.
FIG. 12 illustrates a plot of breathing rate over time with subject movement, according to some aspects of the present disclosure.
FIG. 13 illustrates a plot of breathing rate using a frequency-based technique methods with subject movement.
FIG. 14 illustrates a plot of breathing rate in an empty room, according to some aspects of the present disclosure.
FIG. 15 illustrates a plot of breathing rate in an empty room using a frequency-based technique.

## DETAILED DESCRIPTION

The embodiments set forth below represent the necessary information to enable those skilled in the art to practice the embodiments and illustrate the best mode of practicing the embodiments. Upon reading the following description in light of the accompanying drawing figures, those skilled in the art will understand the concepts of the disclosure and will recognize applications of these concepts not particularly addressed herein. It should be understood that these concepts and applications fall within the scope of the disclosure and the accompanying claims.

It will be understood that, although the terms first, second, etc. may be used herein to describe various elements, these elements should not be limited by these terms. These terms are only used to distinguish one element from another. For example, a first element could be termed a second element, and, similarly, a second element could be termed a first element, without departing from the scope of the present disclosure. As used herein, the term "and/or" includes any and all combinations of one or more of the associated listed items.

The terminology used herein is for the purpose of describing particular embodiments only and is not intended to be limiting of the disclosure. As used herein, the singular forms "a," "an," and "the" are intended to include the plural forms as well, unless the context clearly indicates otherwise. It will be further understood that the terms "comprises," "comprising," "includes," and/or "including" when used herein specify the presence of stated features, integers, steps, operations, elements, and/or components, but do not preclude the presence or addition of one or more other features, integers, steps, operations, elements, components, and/or groups thereof.

Unless otherwise defined, all terms (including technical and scientific terms) used herein have the same meaning as commonly understood by one of ordinary skill in the art to which this disclosure belongs. It will be further understood that terms used herein should be interpreted as having a meaning that is consistent with their meaning in the context of this specification and the relevant art and will not be interpreted in an idealized or overly formal sense unless expressly so defined herein. Additionally, like reference numerals denote like features throughout specification and drawings.

It should be appreciated that the blocks in each diagram or flowchart and combinations of the diagrams or flowcharts may be performed by computer program instructions. Since the computer program instructions may be equipped in a processor of a general-use computer, a special-use computer or other programmable data processing devices, the instructions executed through a processor of a computer or other programmable data processing devices generate means for performing the functions described in connection with a block(s) of each diagram or flowchart. Since the computer program instructions may be stored in a computer-available or computer-readable memory that may be oriented to a computer or other programmable data processing devices to implement a function in a specified manner, the instructions stored in the computer-available or computer-readable memory may produce a product including an instruction for performing the functions described in connection with a block(s) in each diagram or flowchart. Since the computer program instructions may be equipped in a computer or other programmable data processing devices, instructions that generate a process executed by a computer as a series of operational steps are performed by the computer or other programmable data processing devices and operate the computer or other programmable data processing devices may provide steps for executing the functions described in connection with a block(s) in each diagram or flowchart. Additionally, or alternatively, a computer-readable medium can include transient media such as carrier waves and transmission media.

Each block may represent a module, segment, or part of a code including one or more executable instructions for executing a specified logical function(s). Further, it should also be noted that in some replacement execution examples, the functions mentioned in the blocks may occur in different orders. For example, two blocks that are consecutively shown may be performed substantially simultaneously or in a reverse order depending on corresponding functions.

Hereinafter, embodiments are described in detail with reference to the accompanying drawings. Further, although a breathing monitoring system is described in connection with embodiments, as an example, the embodiments may also apply to other monitoring systems with similar technical background or features. Further, embodiments may be modified in such a range as not to significantly depart from the scope of the present disclosure under the determination by one of ordinary skill in the art and such modifications may be applicable to other systems in addition to radar.

Ultrawide-band (hereinafter "UWB") may refer to a short-range high-rate wireless communication technology using a wide frequency band of several GHz or more, low spectral density, and short pulse width (e.g., 1 nsec to 4 nsec) in a baseband state. UWB may mean a band itself to which UWB communication is applied. UWB may enable secure and accurate ranging between devices. Thus, UWB enables relative position estimation based on the distance between two devices or accurate position estimation of a device based on the distance

Furthermore, the following abbreviations may be used throughout: "CIR" for channel impulse response, "CIRE" for channel impulse response estimation, "RFRI" for radar frame repetition interval, "BPM" for breaths per minute, and "STFT" for short time Fourier transform.

Embodiments of the present disclosure provide systems and methods for breath tracking.

Embodiments of the present disclosure provide systems and methods for breath detection.

Embodiments of the present disclosure provide systems and methods for determining a breathing rate.

Embodiments of the present disclosure provide systems and methods for reducing or eliminating false detections and/or lost breathing signals.

The disclosed systems and methods can facilitate several improvements. For example, disclosed systems and methods for breath tracking are more robust, including as to false detections and lost breathing signals. For example, disclosed systems and methods for breath tracking are more robust to irregularities in the breathing periodicity due to a subject's movements and/or unusual breathing pattern. For example, disclosed systems and methods for breath tracking can quickly adapt identify switches between true positive and true negative conditions. For example, disclosed systems and method reduce resource consumption compared to traditional frequency transform-based techniques. In some embodiments described herein, the systems and methods may be well adapted to detect short apneas.

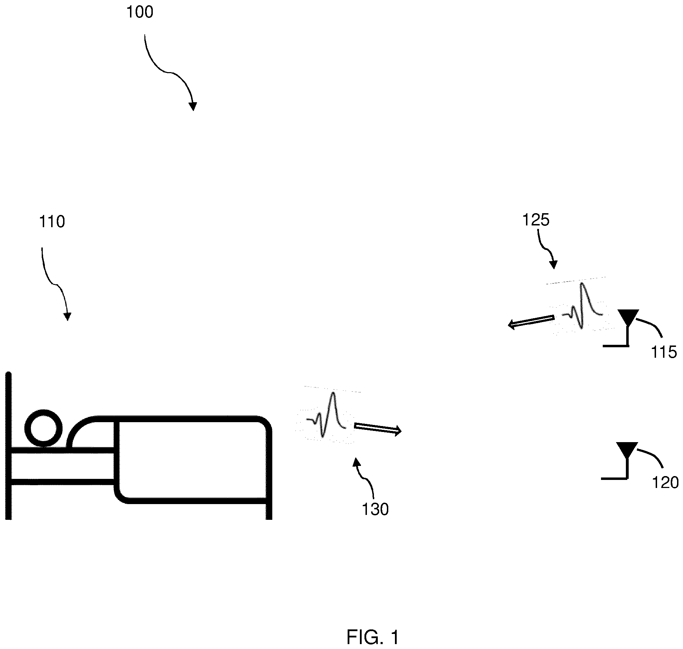

FIG. 1 illustrates a monitoring scenario 100 using radar pulses. In some embodiments, a monitoring scenario 100 includes a subject 110, transmitter 115, and receiver 120. A subject 110 may breathe at a rate which may change over time. While the subject 110 is breathing a transmitter 115 may generate pulses at least a portion of which propagate toward the subject 110. The subject's body may reflect at least some portion of the transmitted pulse 125. The breathing of the subject 100 may introduce a phase difference in the signal, which may be measured at the receiver 120. The receiver 120 may receive a pulse 130 scattered off the subject's chest. For example, a personal mobile device may be placed next to a person's bed at night to track their breathing rate by transmitting and receiving pulses and performing breath monitoring through the systems and methods as described herein.

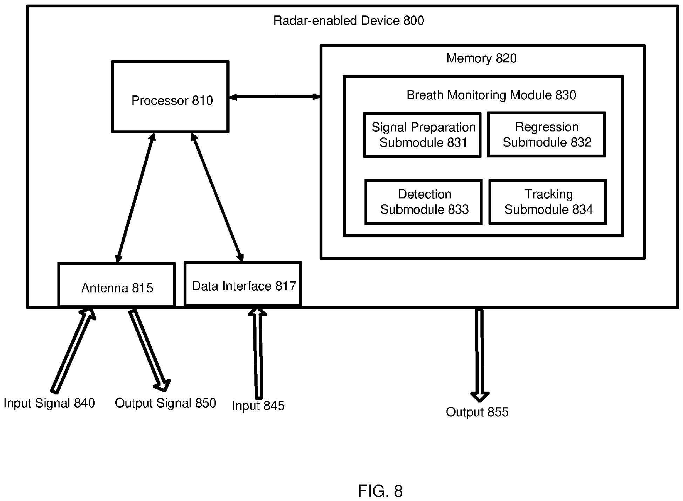

In some embodiments, a transmitter 115 may be contained in a device such a mobile device, medical equipment, and/or the like (e.g. similar to radar-enabled device 800 depicted in, and described with respect to, FIG. 8). The device may be UWB-enabled such that the transmitted pulse 125 is an UWB pulse. The device may be radar-enabled, such that the transmitted pulse 125 is a radar pulse. In some embodiments, a pulse may be structured as a frame according to standard, e.g., FiRa. Similarly, a second device may comprise a receiver 120. The second device may be UWB-enabled such that it is configured to receive and decode a transmitted UWB pulse 125. The second device may be radar-enabled, such that the received pulse 130 comprising a radar pulse may be received. In some embodiments, both the transmitter 115 and receiver 120 may be contained in the same device.

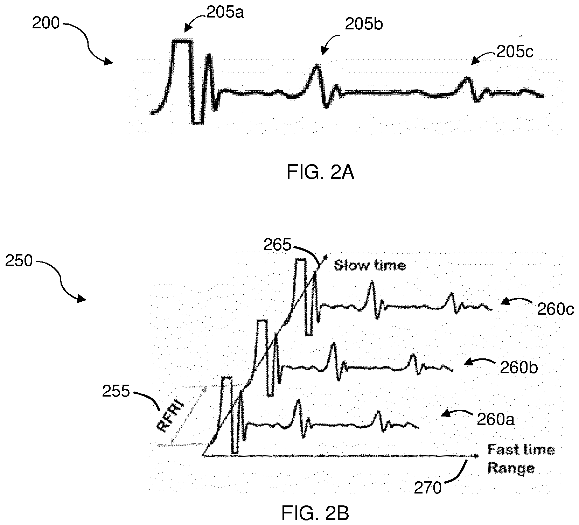

FIGS. 2A and 2B depict graphs of channel impulse responses. FIG. 2A depicts channel impulse response ("CIR") 200. A channel impulse response 200 represents a receiver's response to a received pulse, e.g., the response of receiver 120 to received pulse 130 in FIG. 1. A channel impulse response 200 may comprise a plurality of path responses 205(a)-(c). Each path response may correspond to a delay based on the path a signal took before arriving at a receiver. In the example depicted in FIG. 2A, there are three path responses that comprise the channel impulse response of the receiver over time. In some embodiments, the channel impulse response is a digital estimate ("CIRE") of a continuous channel impulse response. References herein to channel impulse response should be understood to include CIRE, e.g., digital channel impulse responses, and vice-versa, where appropriate. By way of example, the first path response 205a of channel impulse response 200 may correspond to a pulse from a transmitter traversing a straight-line path between the transmitter and receiver; the second path response 205(b) of the channel impulse response 200 may correspond to a pulse from a transmitter traversing a path from a transmitter to a target, e.g. subject 110, and then to a receiver; and the third path response 203(c) of channel impulse response may correspond to a much longer path, such a one describing scattering off walls or other objects in a room. FIG 2A is merely illustrative, there may be more or fewer path responses comprising the channel impulse response, and the shape of the channel impulse response may differ based on a number of factors, including the physical configuration of a receiver, the environment through which a pulse travels, and/or the kinds of objects in the environment that reflect a pulse.

FIG. 2B depicts a graphical representation 250 of a sequence of channel impulse responses or (CIREs) 260a-c resulting from a sequence of transmitted pulses by a transmitter with a specified radar frame repetition interval (RFRI) 255. In other words, there is one CIRE per frame and each frame is comprised by a sequence of pulses. In some embodiments, as described in FIG. 1, the transmitter 115 transmits sequence of pulses 125 at regularly spaced time intervals equal to the RFRI and the receiver generates channel impulse responses 260a-c, one for each transmit sequence of pulse 125. Sometimes the RFRI 255 will be referred to as a frequency. In some embodiment the slow time axis 265 measures the time between transmitted pulses. In some embodiments, the fast time axis 270 corresponds to a delay or range of the various paths taken by the transmitted pulse, such as the length of the path from the transmitter to the subject 110 and the subject to the receiver. Each of the paths of results in a path response, e.g., one of 205a-c, in the channel impulse responses 260a-c. In some embodiments the fast time axis 270 corresponds to a delay measured from the time when each pulse was transmitted by the transmitter. Heuristically, the signal for a path response may vary periodically as a function of the slow time, representing the periodicity of the breathing of the subject in a room. The channel impulse responses depicted in FIGS. 2A-2B are merely illustrative. In some embodiments, the signal that is processed by the breath monitoring system is a digital signal.

The signal as measured by the receiver may be represented mathematically as $r(\tau, kT_s)$ representing the k-th tap of the CIR measured at time $\tau$ at sampling rate $T_s$, where the sampling rate may, in some embodiments, may be between 500MHz and 10GHz. In some embodiments, $r(\tau, kT_s)$ is a complex number. Let K be the number of complex taps of the CIRE. In some embodiments, to deal independently with the real and the imaginary parts of each CIRE tap, the following 2K real CIRE signals are defined

$$r_{2k}(\tau) = Re\big(r(\tau, kT_s)\big) \hspace{4cm} (1)$$

$$r_{2k+1}(\tau) = Im\big(r(\tau, kT_s)\big)$$

Thus, each CIR contains 2K real coefficients: K from the real part and K from the imaginary part. In a static environment with a regular breathing target, the real signals of each CIRE may be given by:

$$r_l(\tau) = \alpha_l p(\tau) + C_l + w_l(\tau), \hspace{4cm} (2)$$

where the function $p(\tau)$ is a normalized periodic function of period A. The function $p(\tau)$ is characteristic of the breathing pattern of a given target at a given moment. It may be sinusoidal but most of the time the inspiration and expiration operations not being symmetrical, it follows a non-sinusoidal periodic pattern. The coefficients $\alpha_l$ are real, representing the amplitude of the oscillations of each part (Re or Im) of the kth tap. In some implementations, the breathing impacts significantly only some of the CIRE coefficients. Thus, most of the $\{\alpha_l\}_l$ may be null. Here $\{\alpha_l\}_l$ are assumed constant over the time but, in practice, they vary either slowly or suddenly if the breathing target has moved for instance. The constant $C_l$ represents the clutter effects which by definition are independent of $\tau$. The function $w_l(\tau)$ is the noise, accounting for effects in both the environment and the receiver. The noise may be stationary over the fast time, but its power may depend on the tap index, $E_\tau\left[\|w_l(\tau)\|^2\right] = \sigma_l^2.$ The signals with the clutter removed may be written

$$c_l(\tau) = r_l(\tau) - C_l = \alpha_l p(\tau) + w_l(\tau), \hspace{3cm} (3)$$

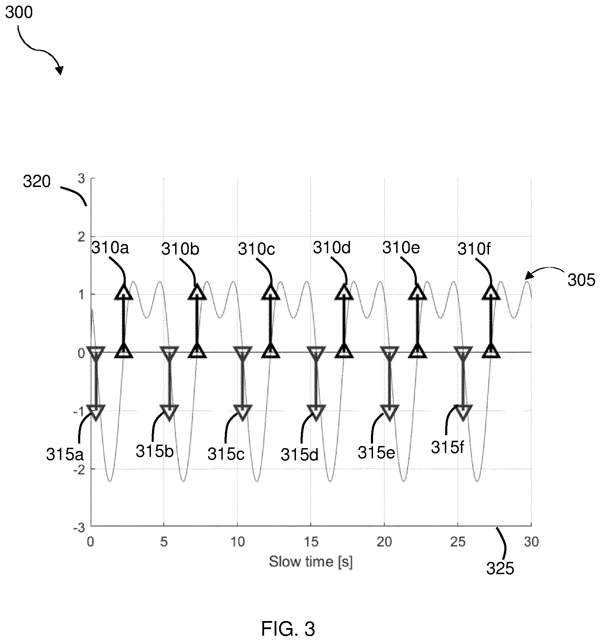

FIG. 3 depicts a graph 300 of an example periodic signal 305. The periodic signal 305 may correspond to $p(\tau)$ as described herein and shown in Eq. 3. Markers 310a-f and 315 a-f identify positions where a periodic signal crosses zero. The vertical axis 320 corresponds to the signal strength in arbitrary units. The horizontal axis 325 corresponds to the slow time, e.g., similar to 265 in FIG. 2B. In some embodiments, by tracking the zeros of a periodic signal a breathing rate may be calculated. For reasons that are described herein, zero-crossings (ZCs) may be distinguished based on whether the periodic signal is increasing or decreasing at the ZC, i.e., is the slope of the graph positive or negative. the direction of ZC way be called the ZC way, i.e., up (increasing) or down (decreasing). Up arrows, marked above zero, are where the periodic function is increasing at a ZC, and down arrows, marked below zero, are where the periodic function is decreasing at a ZC. Thus, FIG. 3 depicts an example of $p(\tau)$ over the slow time $\tau$. Under certain conditions, the delay between 2 successive ZC times going in the same way is proportional to the periodic signal period A. In some embodiments, the systems and methods described herein estimate the ZC time and direction of the ZC of the function $p(\tau)$, and, from this, determine a breathing rate estimate by tracking the time interval between consecutive ZC events.

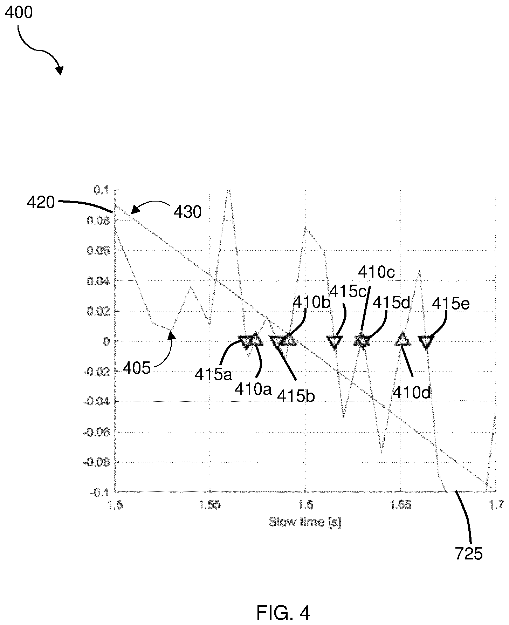

FIG. 4 depicts a graph 400 of an example noisy periodic signal 405. The noisy periodic signal 405 may correspond to the clutter free signal $c_l(\tau)$ as described herein and shown in Eq. 3. As depicted in FIG. 4, up and down markers 410a-d and 415a-e identify positions where a periodic signal crosses zero. The vertical axis 420 corresponds to the signal strength in arbitrary units. The horizontal axis 425 corresponds to the slow time, e.g., similar to 265 in FIG. 2B. In some embodiments, by tracking the zeros of a periodic signal a breathing rate may be calculated. As depicted, noise causes multiple zero-crossings (ZCs) near a ZC of the linear regression line 430. In some embodiments, a ZC for the periodic signal without noise will be referred to as a true ZC. FIG. 4 depicts a regression line 430 that can be used to approximate the true ZC. For reasons that are described herein, ZCs may be distinguished based on whether the period signal is increasing or decreasing at the ZC, i.e., is the slope of the graph positive or negative. Up markers 410a-d mark where the slope of the graph is positive at a ZC and down markers 415a-e mark where the slope of the graph is negative at a ZC.

For a short period of time $[\tau_s, \tau_e]$ around a $p(\tau)$'s ZC event, $p(\tau)$ can be approximated by a straight line. Thus, linear regression may be applied to input signals to estimate ZC times. The time where the straight line resulting from the linear regression crosses the zero level is an estimate of the $p(\tau)$'s ZC time and the sign of the slope of the straight line gives the ZC way.

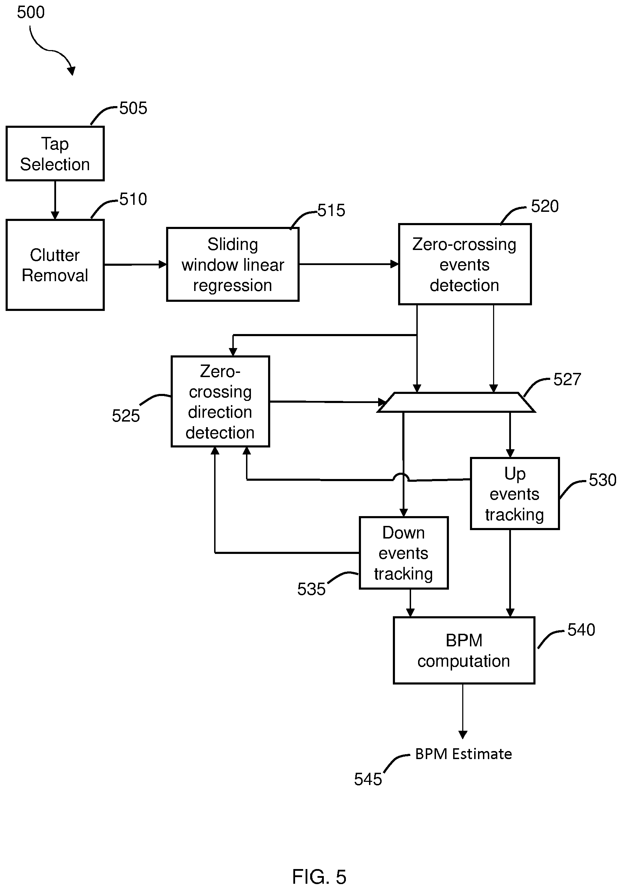

FIG. 5 illustrates a block diagram of a breath monitoring system 500, according to some aspects of the present disclosure. Breath monitoring system 500 may include a tap selection block 505, clutter removal block 510, regression block 515, zero-crossing event detection block 520, zero-crossing direction detection block 525, up tracking block 530, down tracking block 535, and BPM computation block 540. In some embodiments the input to breath monitoring system is the digitized CIR estimation and one or more user provided parameters as described herein. In some embodiments, the breath monitoring system receives the CIR estimation as K complex taps at a regular period (RFRI) $\Delta$. For sake of description, we call $r(m, k)$ the kth complex tap at time $\zeta(m) = \Delta m$. Where $k$ is the fast time index and $m$ is the slow time index. For sake of simplicity, the period $\Delta$ is omitted and assumed to be equal to one, which amounts to a change of units. In some embodiments, the final output of a breath monitoring system, is a breathing rate estimate 545. For example, when monitoring a human subject, the breathing rate estimate may fluctuate around 15 breaths per minute.

In some embodiments, tap selection block 505 selects the taps from the K complex taps of the CIR estimation. Tap selection block 505 may organize the tap signals into a vector expressed as:

$$r(m) = \begin{bmatrix} real(r(k_{st})) \\ imag(r(k_{st})) \\ \vdots \\ \vdots \\ real\left(r(k_{sp})\right) \\ imag\left(r(k_{sp})\right) \end{bmatrix} \tag{4}$$

where $k_{st}$ and $k_{sp}$ are the first and last taps for selection. The vector of Eq. 4 has dimension $L = 2(k_{sp} - kst + 1)$. In some embodiments, a user may provide $k_{st}$ and $k_{sp}$ as input to the breath monitoring system 500. In some embodiments, taps with the highest signal-to-noise ratio (SNR) may be selected.

In some embodiments, a clutter removal block 510 removes the constant background clutter (e.g., $C_j$ in Eq. 2) due to unwanted echoes on the static environment. Clutter removal is applied independently on each component of the vector of K complex taps in Eq. 4, a component may be referred to as $r_l, l \in 1, \dots, L$. Clutter may first be estimated according to the following equation:

$$dc_l(m) = \left(1 - \mu(m)\right)dc_l(m-1) + \mu(m)r_l(m) = dc_l(m-1) + \mu(m)\left(r_l(m) - dc_l(m-1)\right) \tag{5}$$

where $\mu(m)$ is an adaptive algorithm step size or learning rate, which determines the convergence speed. Larger values of $\mu$ result in faster convergence but a larger clutter estimation noise. In some embodiments, $\mu$ may be constant. In some embodiments, $\mu$ may be vary with the slow time, m. In some embodiments, $\mu(m) = \max(\mu_0, \frac{1}{m})$, where $\mu_0$ is a user-defined parameter which ensures that the convergence speed remains sufficiently high. The output of the clutter removal block 510 is an impulse response from only the target's echoes (e.g., only those pulse that reflect off the subject 110 in FIG. 1).

$$c_l(m) = r_l(m) - dc_l(m)$$

This is one example of a method for estimating the clutter; other estimation techniques are contemplated, e.g., stochastic gradient descent.

In some embodiments, a regression block 515 allows regressions to be calculated in a sliding window for taps selected by the tap selection block 505. In some embodiments, the regression block 515 receives the output of clutter removal block, including the clutter free signals.

The set $\{\zeta(i)\}$ may be the sequence of all the ZC times of $p(\tau)$. In the vicinity of the u-th ZC time, $\zeta(u)$, $c_l(\tau)$ can be approximated by

$$c_l(\tau) = \tilde{\alpha}_l(\tau - \zeta_u) + w_l(\tau) = a\tau + b + w_l(\tau) \tag{6}$$

In some embodiments, $N + M + 1$ measures of the clutter free signals are obtained at times $\tau = \{\xi(m-N), \dots, \xi(m + M)\}$ around $\zeta(u)$. If the linear approximation is valid for those measures, the following matrix formulation can be used:

$$c_l(m) = \begin{bmatrix} c_l\left(\xi(m-N)\right) \\ \vdots \\ c_l\left(\xi(m+M)\right) \end{bmatrix} = \begin{bmatrix} \xi(m-N) & 1 \\ \vdots & \vdots \\ \xi(m+M) & 1 \end{bmatrix}\begin{bmatrix} a_l(m) \\ b_l(m) \end{bmatrix} + \begin{bmatrix} w_l\left(\xi(m-N)\right) \\ \vdots \\ w_l\left(\xi(m+M)\right) \end{bmatrix} = \Theta\begin{bmatrix} a_l(m) \\ b_l(m) \end{bmatrix} + w_l(m) \tag{7}$$

An MSE estimator of $a_l(m)$ and $b_l(m)$ may be computed using the following equation:

$$\begin{bmatrix} \hat{a}_l(m) \\ \hat{b}_l(m) \end{bmatrix} = \Theta(m)^{\#}c_l(m) \tag{8}$$

And $\hat{\zeta}_l(m) = -\frac{\hat{b}_l(m)}{\hat{a}_l(m)}$ is a consistent estimate of (u). The sign of $\hat{a}_l(m)$ estimates the way the zero line has been crossed. Moreover, $\rho_l(m) = |\hat{a}_l(m)|^2$ is good indicator of the estimation quality. It may be closely related to the signal amplitude $a_l$ and thus to the SNR. A mean square error $(m)$ between the input vector $c_l(m)$ and linear regression result can also be used to qualify the linear assumption:

$$\epsilon_l(m) = \frac{1}{N+M+1}\left(c_l(m) - \Theta(m)\begin{bmatrix}\hat{a}_l(m)\\\hat{b}_l(m)\end{bmatrix}\right)^T\left(c_l(m) - \Theta(m)\begin{bmatrix}\hat{a}_l(m)\\\hat{b}_l(m)\end{bmatrix}\right) \qquad (9)$$

In some embodiments, all clutter free signals $c_l(\tau)$ having non-zero oscillation amplitude ($|a_l| > 0$) could be used as input signal to get the $(\tau)$'s ZC events. Each one has a different SNRs depending on the oscillation amplitude. In some embodiment, the clutter free signal with the largest magnitude may be selection; this may be formalized as the following equation:

$$l^* = argmax_l(|\alpha_l|) \qquad (10)$$

In some embodiments, clutter free signal $c_l(\tau)$ with positive SNR may be selected.

The discussion above may be reformulated to accommodate the use of multiple tap signals.

$$C(m) = [c_{l_1}(m) \ldots c_{l_L}(m)] = \Theta(m)\begin{bmatrix}a_{l_1}(m) & \cdots & a_{l_L}(m)\\b_{l_1}(m) & \cdots & b_{l_L}(m)\end{bmatrix} + [w_{l_1}(m) \ldots w_{l_L}(m)] \qquad (11a)$$

$$= \Theta(m)\begin{bmatrix}a(m)\\b(m)\end{bmatrix} + W(m)$$

$$\begin{bmatrix}\hat{a}(m)\\\hat{b}(m)\end{bmatrix} = \Theta(m)^\# C(m) \qquad (11b)$$

where $\Theta^\#(m)$ denotes the pseudoinverse of $\Theta(m)$. Because $(m) = -\zeta(u)a(m)$, a weighted least square estimate of $\zeta(u)$ can be computed as

$$\hat{\zeta}(m) = -\frac{\hat{b}(m)\hat{a}(m)^T}{\hat{a}(m)\hat{a}(m)^T} \qquad (12)$$

As the measures are taken regularly, $\xi(m) = m\Delta$, the linear regression matrix $\Theta(m)$ may be mathematically expressed as:

$$\Theta(m) = \begin{bmatrix}(m-N)\Delta & 1\\ \vdots & \vdots\\(m+M)\Delta & 1\end{bmatrix} = \begin{bmatrix}m\Delta & 0\\ \vdots & \vdots\\ m\Delta & 0\end{bmatrix} + \begin{bmatrix}-N\Delta & 1\\ \vdots & \vdots\\ M\Delta & 1\end{bmatrix} = \begin{bmatrix}m\Delta & 0\\ \vdots & \vdots\\ m\Delta & 0\end{bmatrix} + \Theta(0) \qquad (13)$$

and

$$\Theta(0)^\# \Theta(m) = \begin{bmatrix}0 & 0\\ m\Delta & 0\end{bmatrix} + I_{2x2} \qquad (14)$$

Thus, the linear regression results can then be obtained always applying the pseudo inverse of the matrix (0) and then shifting the results as a function of m. The pseudoinverse matrix coefficients can be pre-computed as they only depend on the fixed parameters M and N.

A set of quality indicators for the multi-tap signal regression may be mathematically represented as:

$$\rho(m) = \frac{1}{L}\hat{a}(m)\hat{a}(m)^T \qquad (15a)$$

$$\epsilon(m) = \frac{1}{(N+M+1)L} \sum_{l=1}^{L} \left( c_l(m) - \Theta(m) \begin{bmatrix} \hat{a}_l(m) \\ \hat{b}_l(m) \end{bmatrix} \right)^T \left( c_l(m) - \Theta(m) \begin{bmatrix} \hat{a}_l(m) \\ \hat{b}_l(m) \end{bmatrix} \right) \tag{15b}$$

In some embodiments, the regression described above may be applied continuously as a sliding window process: a ZC time estimate $\hat{\zeta}(m)$ is computed for each input vector $c_l(m)$. When $(m)$ is close to one of the $\zeta(i)$, the linear assumption is valid and $\hat{\zeta}(m)$ shall be stored otherwise $\hat{\zeta}(m)$ is not valid and must be rejected. In some embodiments, the rejection criteria are based on the following observations: (1) It is very likely that $\hat{\zeta}(m)$ is close from $(m)$ only if $\zeta(m)$ is close to one of the $\zeta(i)$; and (2) If $\zeta(m)$ is close to one of the $\zeta(i)$ then the $c_l(m)$'s slopes are not close to 0 and thus $\rho(m)$ should be larger than a given threshold.

Generally, multiple ZC time estimates $\hat{\zeta}(m)$ will be generated for each true value $\zeta(i)$. The estimates corresponding to the same $(i)$ may be identified and averaged to try to get one ZC time estimation per $\zeta(i)$. The details of the event detection of zero-crossings are further discussed with respect to the zero-crossing event detection block 520, below. In some embodiments, the process of regression block 515 may be:

1. Form the $(M + N + 1) \times L$ matrix $C(m)$

$$C(m) = \begin{bmatrix} c_{i_1}(m) & \cdots & c_{i_L}(m) \end{bmatrix} = \begin{bmatrix} c_{i_1}(m - N) & \cdots & c_{i_L}(m - N) \\ \vdots & & \vdots \\ c_{i_1}(m + M) & \cdots & c_{i_L}(m + M) \end{bmatrix} \tag{16}$$

2. Apply the pseudo-inverse of $\Theta(0)$

$$\begin{bmatrix} u(m) \\ v(m) \end{bmatrix} = \begin{bmatrix} u_{i_1}(m) & \cdots & u_{i_L}(m) \\ v_{i_1}(m) & \cdots & v_{i_L}(m) \end{bmatrix} = \Theta(0)^* C(m) \tag{17}$$

where $u(m)$ and $v(m)$ are $1 \times L$ vectors.

3. Compute the slope estimate, ZC time estimate, and weight. For the slope estimate, only the input having the largest amplitude must be considered. This may be expressed as the condition that if $|u_l(m)| > T_a$, then $\hat{a}_l(m) = u_l(m)$, otherwise $\hat{a}_l(m) = 0$, where the threshold $T_a$ is computed relatively to the largest value, $T_a = R_a \max_l |u_l|$ with $R_a$ a user-defined parameter. The ZC estimate is computed using

$$\hat{\zeta}(m) = m\Delta - \frac{v(m)\hat{a}(m)^T}{\hat{a}(m)\hat{a}(m)^T} \tag{18}$$

The weight and MSE are computed as follows, respectively:

$$\rho(m) = \frac{1}{\sum_{l=1}^{L} \delta(\hat{a}_l \neq 0)} \hat{a}(m)\hat{a}(m)^T \tag{19a}$$

$$\epsilon(m) = \frac{1}{(N+M+1)\sum_{l=1}^{L} \delta(\hat{a}_l \neq 0)} \sum_{l=1}^{L} \delta(\hat{a}_l \neq 0) \left( \sum_{n=m-N}^{n=m+M} \left( c_l(n) - \hat{a}_l(n)\left( n - \hat{\zeta}(n) \right) \right) \right) \tag{19b}$$

Referring to zero-crossing event detection block 520 and zero-crossing direction detection block, 525 these blocks may be based on the principle of multitarget tracking. Multitarget tracking improves the robustness of systems and methods described herein to irregularities in the breathing sequence due to, for instance, a subject's movements.

In some embodiments, a zero-crossing event detection block 220 identifies a ZC estimates $\hat{\zeta}(m)$ that corresponds to the true ZC $\zeta(u)$. In some embodiments, this is achieved by adding to a list the successive ZC estimates $\hat{\zeta}(m)$ while they are close to the previous ones. Once this closeness condition is not verified anymore, the list is closed and empty, and the elements that were stored in list are used to compute the ZC detector outputs. The ZC detector outputs are indexes by $j$. The zero-cross event detection block 220 includes original confidence criteria which makes the system and method robust against false breathing detection and wrong estimation. The zero-crossing event detection block may output a ZC time $Z(j)$; a ZC slope vector $A(j)$; and quality indicator/weight $W(j)$. In some embodiments, the weight $W(j)$ is determined from quality indicators computed from $(m)$ and $\epsilon(m)$. When the weight is too low the event may be automatically rejected.

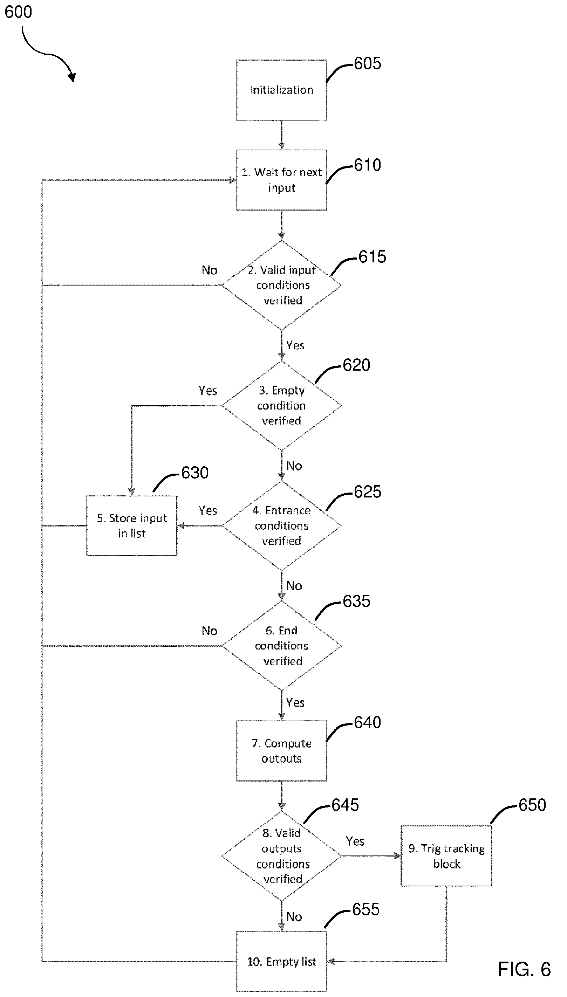

FIG. 6 illustrates a block flow diagram for detecting zero-crossing events. Though FIG. 6 illustrates a number of steps in a sequential order, addition steps may be added and the steps as shown reordered. For the sake of the following description, $\mathcal{L}$ is a list and $j$ is the index for the output of the zero-cross detection block 520. In some embodiments, the system may refer to the radar-enabled computing device as described in FIG. 8.

At step 605, the system is initialized, $\mathcal{L}$ is empty and $j = 1$.

At step 610, the system waits for the next input. In some embodiments, the regression block 515 provides one input per slow time index m. Each input contains four pieces of information $\zeta(m)$, $\hat{a}(m)$ $\rho(m)$, and $\varepsilon(m)$.

At step 615, the system checks input validity conditions. An input is considered only if $\rho(m) > T_r$ and $m - D_r < \zeta(m) < m + D_r$. Where $T_r$ and $D_r$ are user defined paramters.

At step 620, the system checks if the list $\mathcal{L}$ is non-empty.

At step 625, the system check list entrance conditions. An input (i.e., a zero-crossing) can be added to the list $\mathcal{L}$ only if $\min_{n \in \mathcal{L}} |\hat{\zeta}(m) - \hat{\zeta}(n)| < D_l$. That is a new zero-crossing must be sufficient close to an existing zero-crossing in $\mathcal{L}$.

At step 630. the system stores the input in the list, i.e., add the current zero-crossing time index m to the list $\mathcal{L}$.

At step 635, the system check end conditions. In some embodiments the list will be closed when no new elements have added during the last $T_e$ times.

At step 640, outputs may be generated from elements stored in the list $\mathcal{L}$. In some embodiments a ZC time estimate $Z(j)$ is computed, $Z(j) = \text{mean}_{n \in \mathcal{L}} \hat{\zeta}(n)$; a ZC slope vector $A(j)$ is computed, $A(j) = \hat{a}(n^*)$ where $n^* = \text{argmax}_{n \in \mathcal{L}} \rho(n)$; a weight $W(j)$

$$E(j) = \text{mean}_{n \in L} \epsilon(n) \tag{20a}$$

$$R(j) = \text{mean}_{n \in L} \sqrt{\rho(n)} \tag{20b}$$

$$W(j) = \frac{R(j)}{\sqrt{E(j)}} \tag{20c}$$

At step 645, the system checks output validity conditions. In some embodiments, the outputs are considered valid and to trigger the tracking block if: (a) the list contained more than $N_l$ elements and $W(j) > T_o$, where $N_l$ and $T_o$ are user defined parameters.

At step 650 the system triggers the tracking block if the output validity conditions are met.

At step 655, the system empties the list $\mathcal{L}$ and increments the output injex $j = j + 1$.

Referring to up events tracking block and down events tracking blocks 530 and 535, these blocks implement multi-target tracking to separately track zero-crossings with positive and negative slopes, up events and down events, respectively. In the discussion which follows, subscript/superscript references to up (u) or down (d) are suppressed because each block 530, 535 has similar processing steps.

A periodic signal crosses the zero line twice per period: one time in the up direction and one time in the down. At a given ZC time, some inputs may go up while others go down. But then, the opposite will occur at the next ZC time. For sake of simplicity, we will call one of these ZC per period "up" and the other ones "down", independently of the true values of the input signals as they might go in opposite ways.

The delay between an up and a down ZC event may be different from the delay between a down and an up. For example, respiration may not be symmetric in time between exhaling and inhaling. Thus, up and down ZC estimates are processed independently to get 2 period estimates which be combined at the end of the process for a final estimate.

When a new event occurs, one must first identify its "up" or "down" direction. This is achieved by comparing the slope vector $A(j)$ of this new event with some reference vectors $\boldsymbol{D}_u$ and $\boldsymbol{D}_d$ computed by respectively the up and the down tracking blocks.

In practice, the $\zeta(u)$ will not be regularly spaced and from time to time they can disappear during some time if, for instance, the subject is moving. To react quickly to all these changes, the processes described herein is uses multitarget tracking which makes it robust to irregularities in the true sequence.

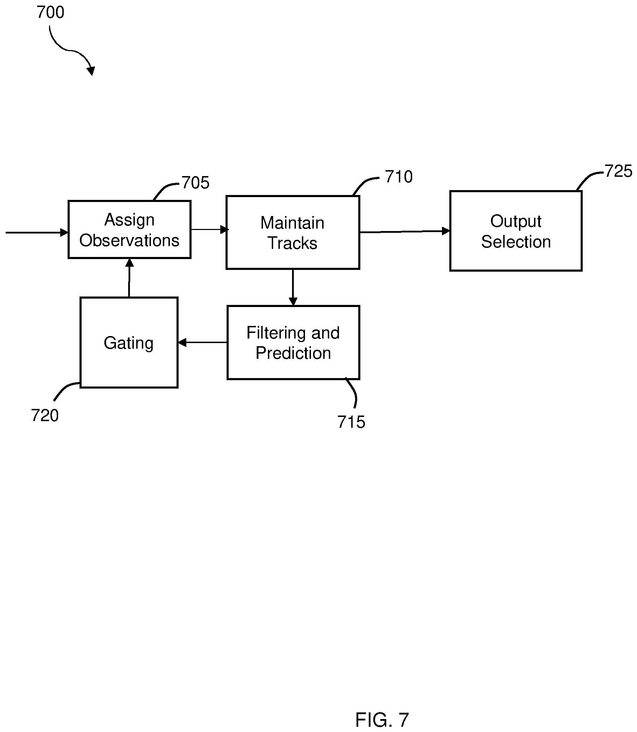

FIG. 7 illustrates a block diagram for events tracking, according to some aspects of the present disclosure. In some embodiments, events tracking block 700 includes an assigning block 705, a maintenance block 710 a filtering and prediction block 715, a gating block 720, and a selection output block 725. In some embodiments, the events tracking block 700 may consist of tracking the breathing period from the ZC event estimates.

Each track $t^{(u)}$ is characterized by a set of parameters:

| | |
|---|---|
| $STATUS^{(u)}$ | Taking values "tentative", "confirmed", or "deleted" |
| $ASSIGNLIST^{(u)}$ | A vector listing the last assigned indexes |
| $Z^{(u)}$ | The last ZC time measures integrated in this track |
| $Y^{(u)}$ | Before last ZC time measures integrated in this track |
| $A^{(u)}$ | Last slope vector measures integrated in this track |
| $P^{(u)}$ | Current period estimation |
| $D^{(u)}$ | A slope vector estimation |
| $V^{(u)}$ | Variance estimate of the period estimation error |
| $G^{(u)}$ | Current gate margin |

In some embodiments, an assigning block 705 associates observations to tracks. The purpose of this block may be to check if a new observation $Z(j)$ can be assigned to the current living tracks. If there is no living track available, the block is by-passed. Assigning block 705 may comprise a number of steps described below.

First, block 705 may fill a cost matrix M. In some embodiments, as some ZC events are not detected, the gap between the current observation and the last measures might be equal to more than one period. Thus, in the cost matrix, the distance of the current observation to "future" ZC times is also considered. Let $u_n$ be the index of the n-th living track, then

$$M(n,p) = \left| Z^{(u_n)} + (p-1)P^{(u_n)} - Z(j) \right| \qquad 21$$

where $p$ indexes the number of periods.

Second, block 705 finds the minimal value and index of $M(n,p)$, $(n^*,p^*) = argmin_{(n,p)}M(n,p)$

Third, block 705 checks that the selected values comply with gating constraints, i.e., if

$$\left| Z^{(u_{n^*})} + (p^*-1)P^{(u_{n^*})} - Z(j) \right| < min\left( G^{(u_{n^*})}, P^{(u)} \right)$$ , then $Z(j)$ and $A(j)$ are assigned to track $u_{n^*}$

$$Y^{(u_{n^*})} = Z^{(u_{n^*})} \qquad (22a)$$

$$Z^{(u_{n^*})} = Z(j) \qquad (22b)$$

$$A^{(u_{n^*})} = A(j) \qquad (22c)$$

Otherwise, the observation is assigned to none of the existing tracks.

In some embodiments a track maintenance block 710, modifies track and/or creates new tracks. Track maintenance block 710 may comprise a number of steps. First, block 710 may create a new track with unassigned $Z(j)$:

| | |
|---|---|
| $STATUS^{(u)}$ | tentative |
| $ASSIGNLIST^{(u)}$ | = [ ] |
| $Z^{(u)}$ | $Z(j)$ |
| $Y^{(u)}$ | 0 |
| $A^{(u)}$ | $A(j)$ |
| $P^{(u)}$ | $P^{(u-1)}$ <br> Note that $P^{(0)}$ is a user defined value. |
| $D^{(u)}$ | 0 |
| $V^{(u)}$ | 0 |
| $G^{(u)}$ | $P^0$ |

Second, in some embodiments, a tentative track is confirmed if the track has been assigned for at least M4T2C times during the last N4T2C events. M4T2C and N4T2C are user-defined parameters. By default, M4T2C = 2 and N4T2C = 3. Third, a confirmed track is deleted if the track has not been assigned at T4C2D seconds. A tentative track is deleted if the track has not been assigned at T4T2D seconds.

In some embodiments, a filtering and prediction block 715 may be a first order loop filter and applied for the tracking of the period estimation. Block 715 may computer the following:

| Error estimation | $e = P^{(u_{n^*})} - \dfrac{\left(Z^{(u_{n^*})} - Y^{(u_{n^*})}\right)}{p^*}$ |
|---|---|
| Period estimation Update | $P^{(u_{n^*})} = P^{(u_{n^*})} - \lambda_p e$ |
| Error variance estimation update | $V^{(u_{n^*})} = (1 - \delta)V^{(u_{n^*})} + \lambda_V e^2$ |
| Reference slop vector update | $D^{(u_{n^*})} = D^{(u_{n^*})} + \lambda_D\left(D^{(u_{n^*})} - A^{(u_{n^*})}\right)$ |

In some embodiments, a gating block 720 may generate a gating value, used in the assigning block 705. The gating value is computed according to $G^{(u_{n^*})} = T_g \sqrt{V^{(u_{n^*})}}$ , where $T\_g$ is a user-defined scaling factor.

In some embodiments, the selection output block 725, at each time step, may output a period estimate $P_x$ with its confidence estimate $C_x$ and a way vector estimate $D_x$, x = {u,d} depending on which one of the up or down block is considered. At a given time, the tracking block might be maintaining several living tracks, some having a tentative status, some a confirmed one. Let u* be the index of the most recent living confirmed track, if it exists. Then,

$$P_x = P^{(u^*)}$$

$$C_x = 1$$

$$D_x = D^{(u^*)}$$

If there is no living track, then

$$P_x = 0$$

$$C_x = 0$$

$$D_x = 0$$

In some embodiments, the track configuration parameters may be defined in such a way that at a given time only one track can have a confirmed status.

In some embodiments, a zero-crossing direction detection block 525 may identify which one of the up and down tracking blocks must process the last event. Up and down zero-crossing event are tracked independently to make the algorithm robust against non-symmetric breathing patterns. A multi-dimension direction detection solution is proposed. The crossing direction is given by comparing the results of the scalar product of (j) with reference vectors $D_u$ and $D_d$. If $|A(j)^T D_u| > |A(j)^T D_d|$ then $D(j) = Up$, otherwise D(j) = *Down*.

In some embodiments, a BPM computation block 540 may compute a breathing rate estimate 545. The output from up tracking block and down tracking block may be fed into a BPM computation block 540, denoted as $P_u$, $P_d$, $C_u$, and $C_d$, as described above. The following Table shows an estimate for the breathing rate for various confidence estimates $C_u$, and $C_d$.

Table 1

| $C_u$ | $C_d$ | BPM |
|---|---|---|
| 1 | 1 | $(P_u + P_d)/2$ |

(continued)

| $C_u$ | $C_d$ | BPM |
|---|---|---|
| 1 | 0 | $P_u$ |
| 0 | 1 | $P_d$ |
| 0 | 0 | No estimate |

As shown in Table 1, only when both confidence estimates are zero is breathing rate estimate no output. By tracking up and down zero-crossings, the method and systems described herein are capable of estimating the breathing rate even if there is no confidence in one type of zero-crossing, i.e., up or down.

FIG. 8 is a simplified diagram of a radar-enabled device 800. One or more radar-enabled devices 800 may be present in the scenarios depicted in, and described with respect to, FIG. 1, according to embodiments described herein. In some embodiments, a radar-enabled device 800 may carry out breath monitoring systems and methods depicted in, and described with respect to, FIGS. 2-7. As shown in FIG. 8, radar-enabled device 800 includes a processor 810 coupled to memory 820. Operation of radar-enabled device 800 is controlled by processor 810. And although radar-enabled device 800 is shown with only one processor 810, it is understood that processor 810 may be representative of one or more central processing units, multi-core processors, microprocessors, microcontrollers, digital signal processors, field programmable gate arrays (FPGAs), application specific integrated circuits (ASICs), graphics processing units (GPUs) and/or the like in radar-enabled device 800. Radar-enabled device 800 may be implemented as a stand-alone subsystem, as a board added to a computing device, and/or partially or wholly as a virtual machine.

Memory 820 may be used to store software executed by radar-enabled device 800 and/or one or more data structures used during operation of radar-enabled device 800. Memory 820 may include one or more types of machine-readable media. Some common forms of machine-readable media may include floppy disk, flexible disk, hard disk, magnetic tape, any other magnetic medium, CD-ROM, any other optical medium, punch cards, paper tape, any other physical medium with patterns of holes, RAM, PROM, EPROM, FLASH-EPROM, any other memory chip or cartridge, and/or any other medium from which a processor or computer is adapted to read.

Processor 810 and/or memory 820 may be arranged in any suitable physical arrangement. In some embodiments, processor 810 and/or memory 820 may be implemented on a same board, in a same package (e.g., system-in-package), on a same chip (e.g., system-on-chip), and/or the like. In some embodiments, processor 810 and/or memory 820 may include distributed, virtualized, and/or containerized computing resources. Consistent with such embodiments, processor 810 and/or memory 820 may be located in one or more data centers and/or cloud computing facilities.

In some examples, memory 820 may include non-transitory, tangible, machine readable media that includes executable code that when run by one or more processors (e.g., processor 810) may cause the one or more processors to perform the methods described in further detail herein. For example, as shown, memory 820 includes instructions for breath monitoring module 830 that may be used to implement and/or emulate the systems and models, and/or to implement any of the methods described herein. breath monitoring module 830 may receive input signal 840 via the antenna 815 and/or generate an output signal 850. Examples of the input signal may include earlier transmitted pulses that have reflected from objects in the environment of the device, e.g., as depicted in FIG. 1, which generate the estimated channel impulse response as depicted in and described with respect to FIGS. 2A and 2B. The input signal may be structured in frames, which, for example, may take the form as described in any a number of different standards, i.e. IEEE, FiRa, and/or the like. Examples of the output signal may include transmission frames by the radar-enabled device 800 at a particular rate, e.g., the RFRI rate as depicted in, and described with respect to, FIG. 2B.

The antenna 815 may comprise a transceiver, a separate transmitter and receiver, or any other means of transmitting in radar or other signaling modalities applicable to the systems and methods described herein. For example, the radar-enabled device 800 may receive the input signal 840 from an earlier transmitted pulse, e.g., a pulse transmitted by antenna 815.

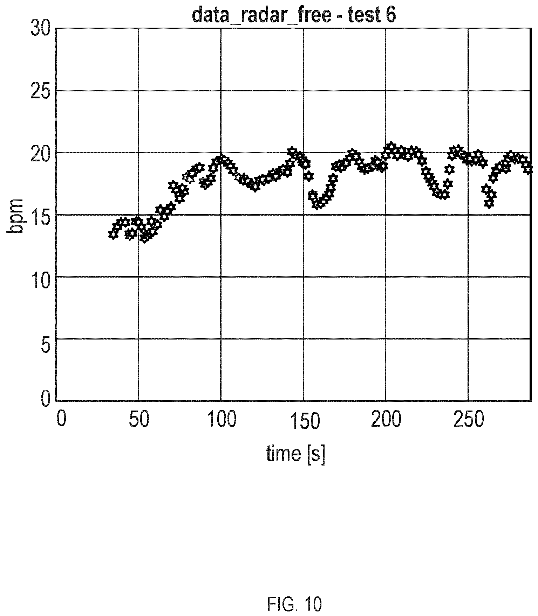

The data interface 817 may comprise a communication interface, a user interface (such as a voice input interface, a graphical user interface, and/or the like). For example, the radar-enabled device 800 may receive the input 845 (such as a set of parameters for breathing monitoring) from a networked database via a communication interface. Or the radar-enabled device 800 may receive the input 845, such as user-specified parameters for breathing monitoring, from a user via the user interface. The radar-enabled device 800 may generate output 855. For example, the output 855 may a numerical value for a breathing rate or a sequence of numerical values over time representing the changes in a breathing rate, e.g., as shown in FIGS. 10, 12, and 14.

In some embodiments, the Breath Monitoring Module 330 is configured to control the content and timing of output signal 855. The Breath Monitoring Module 830 may further include Signal Preparation Submodule 831 (e.g., instructions implementing the tap selection block 505 and/or clutter removal block 510 in FIG. 5), Regression Submodule 832 (e.g., implementing the regression block 515 in FIG. 5), Detection Submodule 833 (e.g., for implementing the zero-crossing events detection block 520 and/or the zero-crossing direction detection block as described in FIG. 5-7), and/or Tracking Submodule 834 (e.g., for implementing blocks 530 and/or 535 as depicted in FIG. 5).

Some examples of UWB devices, such as UWB device 300 may include non-transitory, tangible, machine readable media that include executable code that when run by one or more processors (e.g., processor 310) may cause the one or more processors to perform the processes of method. Some common forms of machine-readable media that may include the processes of method are, for example, floppy disk, flexible disk, hard disk, magnetic tape, any other magnetic medium, CD-ROM, any other optical medium, punch cards, paper tape, any other physical medium with patterns of holes, RAM, PROM, EPROM, FLASH-EPROM, any other memory chip or cartridge, and/or any other medium from which a processor or computer is adapted to read.

In some embodiments, radar-enabled device 800 may be configured for UWB.

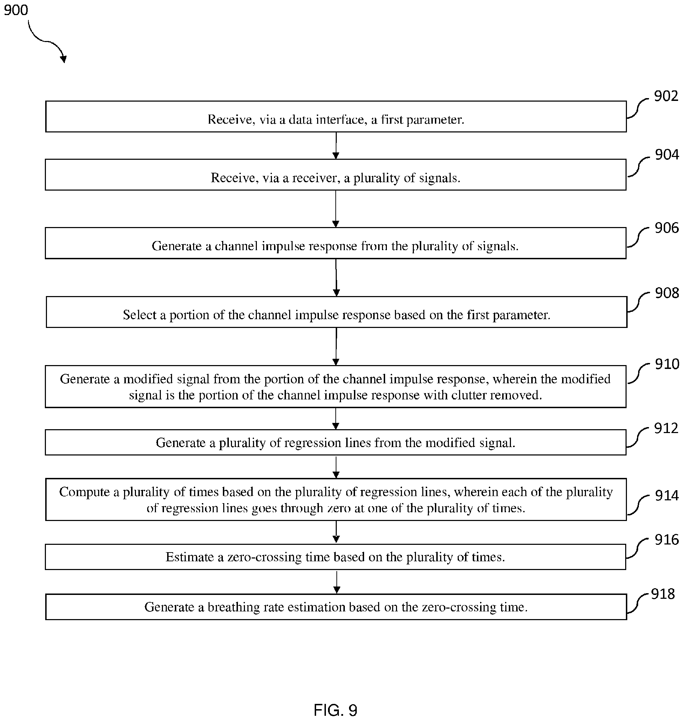

FIG. 9 illustrates an example method for breathing rate estimation and tracking by a radar-enabled device, according to some aspects of the present disclosure. Method 900 is merely an example, and is not intended to limit the present disclosure beyond what is explicitly recited in the claims. Additional operations can be provided before, during, and after the method 900, and some operations described can be replaced, eliminated, or moved around for additional embodiments of FIGS. 1-8. For ease of illustration, FIG. 9 is described in connection with FIGS. 1-8. In some embodiments, method 900 may be implemented by UWB Device 800, as in FIG. 8, or any of the radar-enabled devices described herein.

At step 902, a radar-enabled device (e.g., 800 in FIG. 8) receives, via a data interface (e.g., 817 in FIG. 8), a first parameter (e.g., the number of taps as described herein).

At step 904, a radar-enabled device (e.g., 800 in FIG. 8) receives, via a receiver (e.g., antenna 815 in FIG. 8), a plurality of signals (e.g., 814 in FIG. 8 and as depicted, and described with respect to, FIG. 1).

At step 906, a radar-enabled device (e.g., 800 in FIG. 8) generates a channel impulse response (e.g., as described in FIGS 2A, 2B) from the plurality of signals. In some embodiments, channel impulse response may be a channel impulse response estimate, which may be a digital signal.

At step 908, a radar-enabled device (e.g., 800 in FIG. 8) selects (e.g., 505 in FIG. 5 as implemented by signal preparation submodule 831 in FIG. 8) a portion (e.g., as expressed in Eq. 4) of the channel impulse response based on the first parameter.

At step 910, a radar-enabled device (e.g., 800 in FIG. 8) generates (e.g., using signal preparation submodule 831 in FIG. 8) a modified signal (e.g., as expressed by Eq. 3) from the portion of the channel impulse response, wherein the modified signal is the portion of the channel impulse response with clutter removed (e.g., 510 in FIG. 5). In some embodiments, the clutter may comprise background echoes in an environment (e.g., as depicted in, and described with respect to, FIG. 1).

At step 912, a radar-enabled device (e.g., 800 in FIG. 8) generates (e.g., 515 in FIG. 5 as implemented by regression submodule 832) a plurality of regression lines from the modified signal.

At step 914, a radar-enabled device (e.g., 800 in FIG. 8) computes a plurality of times based on the plurality of regression lines (e.g., 520 in FIG. 5 as implemented by detection submodule 833 in FIG. 8), wherein each of the plurality of regression lines goes through zero at one of the plurality of times.

At step 916, a radar-enabled device (e.g., 800 in FIG. 8) estimates (e.g., 520 in FIG. 5 as implemented by detection submodule 833 in FIG. 8) a zero-crossing time based on the plurality of times. In some embodiments, a radar-enabled device sorts the zero-crossing time based on a direction of change (as determined by 525 in FIG. 5) of the modified signal at the zero-crossing time. In some embodiments, a radar-enabled device tracks estimated zero-crossing times based on a cost matrix, wherein the cost matrix is based on a difference between the estimated zero-crossing time and a zero-crossing time stored in a track. In some embodiments, a radar-enabled device assigns an estimated zero-crossing time to the track if a gating constraint is satisfied. In some embodiments, the radar-enabled device detects a false zero-crossing time based on one or more confidence criteria.

At step 918, a radar-enabled device (e.g., 800 in FIG. 8) generates a breathing rate estimation (e.g., 540 in FIG. 5) based on the zero-crossing time. In some embodiments, the breathing rate estimation may be generated at each of a plurality of time steps. The breathing rate estimation may be output by the radar-enabled device, displayed on a user interface, or otherwise made available.

In some embodiments, further steps of method 900 may include a radar-enabled device transmitting (e.g., 850 in FIG. 8) pulses at a given frequency (e.g., the RFRI 255 as depicted in, and described with respect to, FIG. 2B)

Referring to FIGS. 10-15, plots of a breathing rate are depicted using the systems and methods described herein. Furthermore, heat maps are depicted for the prediction of the breathing rate using a frequency-based method. The frequency-based method used was short time Fourier transform. In FIGS. 10-15, the horizontal axis is the slow time axis in seconds. In the plots of the frequency-based technique the vertical axis represents the frequency bins. In the plots of according to the systems and methods described herein, the vertical axis is in breaths per minute.

FIG. 10 illustrates a plot of breathing rate over time for a stationary subject according to some aspects of the present disclosure . The estimated breathing rate is shown by the individual data points in FIG. 10. While the data was being taken and breathing rate estimated, the subject being measured was not given any particular instruction but to breathe as they normally would. FIG. 10 depicts the variations in breathing rate over time and the uninterrupted estimates of the breathing rate across the entire experiment duration.

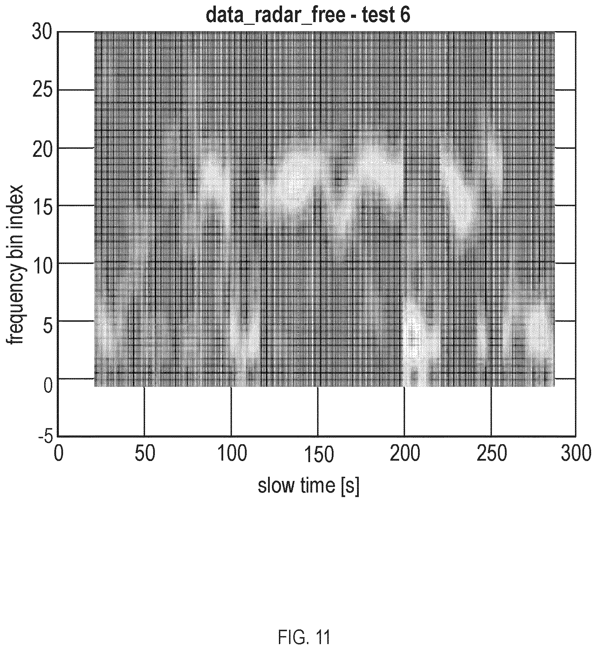

FIG. 11 shows the results from the same subjects breathing as described with respect to FIG. 10, but a frequency-based technique was used. As shown in FIG. 11, while the frequency-based method occasionally has higher intensity in the correct frequency bins (in the 15-20 range), for long stretches of time the frequency-based method is unable to accurately monitor the breathing rate.

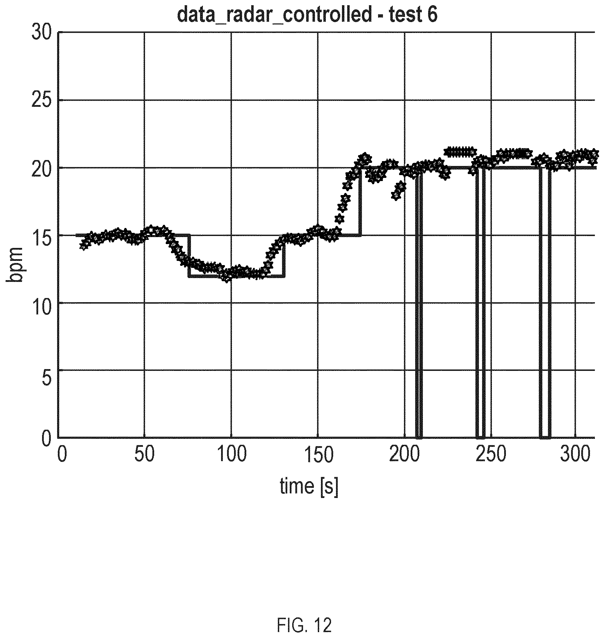

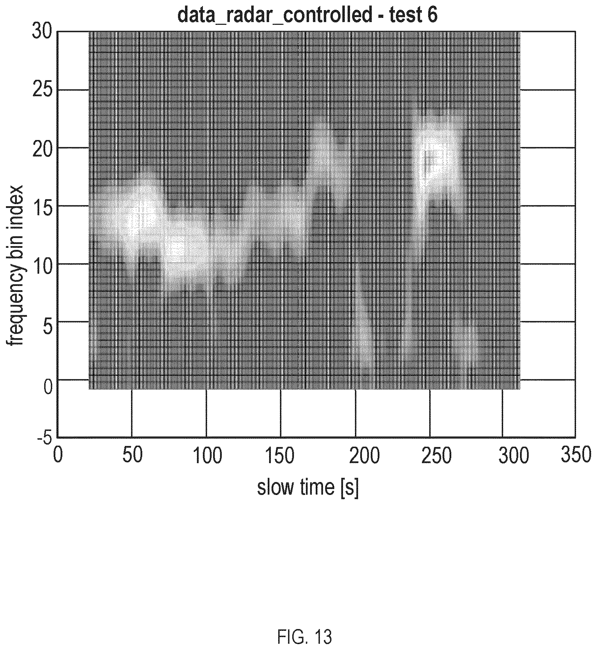

FIG. 12 illustrates a plot of breathing rate over time with subject movement, according to some aspects of the present disclosure. For the experiment plotted in FIGS. 12 and 13 a subject was asked to begin moving their arms at a certain point in time. The experiment was designed to see if the systems and methods as described herein were robust to subject movement. As can be seen in FIG 12. the individual points accurately track the breathing right. The breathing rate around 175 seconds corresponding to the subject beginning to move. Notably, the breathing rate is not lost by the movement of the subject.

FIG. 13 illustrates a plot of breathing rate using a frequency-based technique methods with subject movement. FIG. 13 shows thew frequency-based method tends to perform poorly for subject movement. When the subject begins to move around the 175 second mark, the frequency-based method loses intensity in the correct frequency bins.

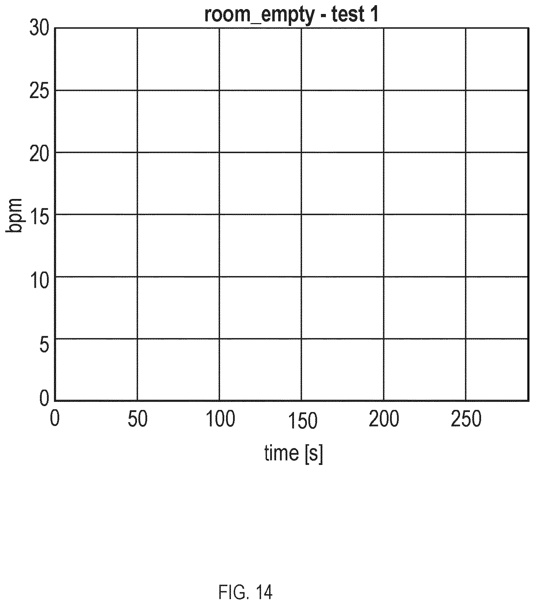

FIG. 14 illustrates a plot of breathing rate in an empty room, according to some aspects of the present disclosure. As shown in FIG. 14, no breathing rate was detected, as would be expected for an empty room.

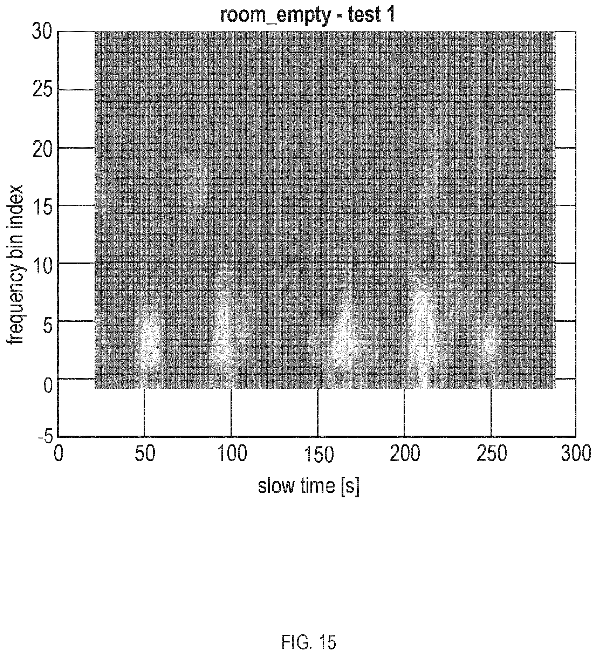

FIG. 15 illustrates a plot of breathing rate in an empty room using a frequency-based technique. Contra to the FIG. 14, the frequency-based method detects spurious frequencies which do not reflect the breathing of any subject.

While some of the terms used herein may match or approximate those of a particular standard, such as FiRa, those skilled in the art will recognize their relevance and application to other protocols based on the concept of ranging round (e.g., as defined in the CCC - Car Connectivity Consortium).

Thus, from one perspective, there have now been described, techniques including methods, systems, and devices for breathing detection and monitoring comprising: receiving, via a data interface, a first parameter; receiving, via a receiver, a plurality of signals; generating a channel impulse response from the plurality of signals; selecting a portion of the channel impulse response based on the first parameter; generating a modified signal from the portion of the channel impulse response, wherein the modified signal is the portion of the channel impulse response with clutter removed; generating a plurality of regression lines from the modified signal; computing a plurality of times based on the plurality of regression lines, wherein each of the plurality of regression lines goes through zero at one of the plurality of times; estimating a zero-crossing time based on the plurality of times; and generating a breathing rate estimation based on the zero-crossing time.

Those skilled in the art will recognize improvements and modifications to the preferred embodiments of the present disclosure. All such improvements and modifications are considered within the scope of the concepts disclosed herein and the claims that follow

Further examples of feature combinations taught by the present disclosure are set out in the following numbered clauses:

1. A method for breathing detection and monitoring comprising:

   receiving, via a data interface, a first parameter;
   receiving, via a receiver, a plurality of signals;
   generating a channel impulse response from the plurality of signals;
   selecting a portion of the channel impulse response based on the first parameter;
   generating a modified signal from the portion of the channel impulse response, wherein the modified signal is the portion of the channel impulse response with clutter removed;
   generating a plurality of regression lines from the modified signal;
   computing a plurality of times based on the plurality of regression lines, wherein each of the plurality of regression lines goes through zero at one of the plurality of times;
   estimating a zero-crossing time based on the plurality of times; and
   generating a breathing rate estimation based on the zero-crossing time.

2. The method of clause 1, further comprising:
sorting the zero-crossing time based on a direction of change of the modified signal at the zero-crossing time.

3. The method of clause 2, further comprising:

   tracking estimated zero-crossing times based on a cost matrix, wherein the cost matrix is based on a difference between the estimated zero-crossing time and a zero-crossing time stored in a track; and

assigning the estimated zero-crossing time to the track if a gating constraint is satisfied.

4. The method of any preceding clause, wherein the channel impulse response is a channel impulse response estimate.

5. The method of any preceding clause, wherein the breathing rate estimation is generated at each of a plurality of time steps.

6. The method of any preceding clause, further comprising:
detecting a false zero-crossing time detection based on one or more confidence criteria.

7. The method of any preceding clause, wherein the clutter comprises background echoes in an environment.

8. A device, comprising:

a receiver;
a memory storing instructions; and
one or more processors configured to execute the instructions to cause the device to perform operations comprising:

receiving, via a data interface, a first parameter;
receiving, via a receiver, a plurality of signals;
generating a channel impulse response from the plurality of signals;
selecting a portion of the channel impulse response based on the first parameter;
generating a modified signal from the portion of the channel impulse response, wherein the modified signal is the portion of the channel impulse response with clutter removed;
generating a plurality of regression lines from the modified signal;
computing a plurality of times based on the plurality of regression lines, wherein each of the plurality of regression lines goes through zero at one of the plurality of times;
estimating a zero-crossing time based on the plurality of times; and
generating a breathing rate estimation based on the zero-crossing time.

9. The device of clause 8, wherein one or more processors are further configured to execute instructions comprising:
sorting the zero-crossing time based on a direction of change of the modified signal at the zero-crossing time.

10. The device of clause 9, wherein one or more processors are further configured to execute instructions comprising:

tracking estimated zero-crossing times based on a cost matrix, wherein the cost matrix is based on a difference between the estimated zero-crossing time and a zero-crossing time stored in a track; and
assigning the estimated zero-crossing time to the track if a gating constraint is satisfied.

11. The device of any of clauses 8 to 10, wherein the channel impulse response is a channel impulse response estimate.

12. The device of any of clauses 8 to 11, wherein the breathing rate estimation is generated at each of a plurality of time steps.

13. The device of any of clauses 8 to 12, wherein one or more processors are further configured to execute instructions comprising:
detecting a false zero-crossing time detection based on one or more confidence criteria.

14. The device of any of clauses 8 to 13, wherein the clutter comprises background echoes in an environment.

15. A computer-readable medium encoding instructions which, when executed by one or more processors, cause the one or more processors to:

receive, via a data interface, a first parameter;
generate a channel impulse response from a plurality of signals;

select a portion of the channel impulse response based on the first parameter;
generate a modified signal from the portion of the channel impulse response, wherein the modified signal is the portion of the channel impulse response with clutter removed;
generate a plurality of regression lines from the modified signal;
compute a plurality of times based on the plurality of regression lines, wherein each of the plurality of regression lines goes through zero at one of the plurality of times;
estimate a zero-crossing time based on the plurality of times; and
generate a breathing rate estimation based on the zero-crossing time.

16. The computer-readable medium of clause 15, wherein the instructions, when executed by the one or more processors, further cause the one or more processors to:
sort the zero-crossing time based on a direction of change of the modified signal at the zero-crossing time.

17. The computer-readable medium of clause 16, wherein the instructions, when executed by the one or more processors, further cause the one or more processors to:

track estimated zero-crossing times based on a cost matrix, wherein the cost matrix is based on a difference between the estimated zero-crossing time and a zero-crossing time stored in a track; and
assign the estimated zero-crossing time to the track if a gating constraint is satisfied.

18. The machine-readable medium of any of clauses 15 to 17, wherein the channel impulse response is a channel impulse response estimate.

19. The machine-readable medium of any of clauses 15 to 18, wherein the breathing rate estimation is generated at each of a plurality of time steps.

20. The computer-readable medium of any of clauses 15 to 19, wherein the instructions, when executed by the one or more processors, further cause the one or more processors to:
detect a false zero-crossing time detection based on one or more confidence criteria.

## Claims

1. A method for breathing detection and monitoring comprising:

   receiving, via a data interface, a first parameter;
   receiving, via a receiver, a plurality of signals;
   generating a channel impulse response from the plurality of signals;
   selecting a portion of the channel impulse response based on the first parameter;
   generating a modified signal from the portion of the channel impulse response, wherein the modified signal is the portion of the channel impulse response with clutter removed;
   generating a plurality of regression lines from the modified signal;
   computing a plurality of times based on the plurality of regression lines, wherein each of the plurality of regression lines goes through zero at one of the plurality of times;
   estimating a zero-crossing time based on the plurality of times; and
   generating a breathing rate estimation based on the zero-crossing time.

2. The method of claim 1, further comprising:
   sorting the zero-crossing time based on a direction of change of the modified signal at the zero-crossing time.

3. The method of claim 2, further comprising:

   tracking estimated zero-crossing times based on a cost matrix, wherein the cost matrix is based on a difference between the estimated zero-crossing time and a zero-crossing time stored in a track; and
   assigning the estimated zero-crossing time to the track if a gating constraint is satisfied.

4. The method of any preceding claim, wherein the channel impulse response is a channel impulse response estimate.

5. The method of any preceding claim, wherein the breathing rate estimation is generated at each of a plurality of time steps.

6. The method of any preceding claim, further comprising:
detecting a false zero-crossing time detection based on one or more confidence criteria.

7. The method of any preceding claim, wherein the clutter comprises background echoes in an environment.

8. A device, comprising:

a receiver;
a memory storing instructions; and
one or more processors configured to execute the instructions to cause the device to perform operations comprising:

receiving, via a data interface, a first parameter;
receiving, via a receiver, a plurality of signals;
generating a channel impulse response from the plurality of signals;
selecting a portion of the channel impulse response based on the first parameter;
generating a modified signal from the portion of the channel impulse response, wherein the modified signal is the portion of the channel impulse response with clutter removed;
generating a plurality of regression lines from the modified signal;
computing a plurality of times based on the plurality of regression lines, wherein each of the plurality of regression lines goes through zero at one of the plurality of times;
estimating a zero-crossing time based on the plurality of times; and
generating a breathing rate estimation based on the zero-crossing time.

9. The device of claim 8, wherein one or more processors are further configured to execute instructions comprising:
sorting the zero-crossing time based on a direction of change of the modified signal at the zero-crossing time.

10. The device of claim 9, wherein one or more processors are further configured to execute instructions comprising:

tracking estimated zero-crossing times based on a cost matrix, wherein the cost matrix is based on a difference between the estimated zero-crossing time and a zero-crossing time stored in a track; and
assigning the estimated zero-crossing time to the track if a gating constraint is satisfied.

11. The device of any of claims 8 to 10, wherein the channel impulse response is a channel impulse response estimate.

12. The device of any of claims 8 to 11, wherein the breathing rate estimation is generated at each of a plurality of time steps.

13. The device of any of claims 8 to 12, wherein one or more processors are further configured to execute instructions comprising:
detecting a false zero-crossing time detection based on one or more confidence criteria.

14. The device of any of claims 8 to 13, wherein the clutter comprises background echoes in an environment.

15. A computer-readable medium comprising instructions which, when executed by a computer, cause the computer to carry out the method of any of claims 1 to 7.

FIG. 1

FIG. 2A

FIG. 2B

FIG. 3

FIG. 4

500

505

Tap
Selection

510

Clutter
Removal

515

Sliding
window linear
regression

520

Zero-crossing
events
detection

527

525

Zero-
crossing
direction
detection

530

Up
events
tracking

535

Down
events
tracking

540

BPM
computation

545 BPM Estimate

FIG. 5

600

FIG. 6

605 — Initialization

610 — 1. Wait for next input

615 — 2. Valid input conditions verified — No

Yes

620 — 3. Empty condition verified — Yes

No

625 — 4. Entrance conditions verified — Yes

630 — 5. Store input in list

No

635 — 6. End conditions verified — No

Yes

640 — 7. Compute outputs

645 — 8. Valid outputs conditions verified — Yes

650 — 9. Trig tracking block

No

655 — 10. Empty list

700

705

Assign
Observations

710

Maintain
Tracks

725

Output
Selection

720

Gating

Filtering and
Prediction

715

FIG. 7

Radar-enabled Device 800

Processor 810

Memory 820

Breath Monitoring Module 830

| Signal Preparation Submodule 831 | Regression Submodule 832 |
| Detection Submodule 833 | Tracking Submodule 834 |

Antenna 815

Data Interface 817

Input Signal 840     Output Signal 850     Input 845

Output 855

FIG. 8

900

Receive, via a data interface, a first parameter. — 902

Receive, via a receiver, a plurality of signals. — 904

Generate a channel impulse response from the plurality of signals. — 906

Select a portion of the channel impulse response based on the first parameter. — 908

Generate a modified signal from the portion of the channel impulse response, wherein the modified signal is the portion of the channel impulse response with clutter removed. — 910

Generate a plurality of regression lines from the modified signal. — 912

Compute a plurality of times based on the plurality of regression lines, wherein each of the plurality of regression lines goes through zero at one of the plurality of times. — 914

Estimate a zero-crossing time based on the plurality of times. — 916

Generate a breathing rate estimation based on the zero-crossing time. — 918

FIG. 9

FIG. 10

data_radar_free - test 6

FIG. 11

data_radar_controlled - test 6

FIG. 12

data_radar_controlled - test 6

FIG. 13

room_empty - test 1

FIG. 14

FIG. 15

# EUROPEAN SEARCH REPORT

**Application Number**

EP 24 21 4533

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2022/373646 A1 (NGUYEN HOANG VIET [US] ET AL) 24 November 2022 (2022-11-24) <br> * abstract; figures 3, 4A, 4D, 5A * <br> * paragraphs [0067] - [0109], [0118], [0125] - [0139] * | 1-15 | INV. <br> G01S7/288 <br> A61B5/0507 <br> A61B5/08 <br> A61B5/00 <br> G01S7/292 <br> G01S7/41 <br> G01S13/02 <br> G01S13/10 <br> G01S13/88 |
| A | US 2023/021342 A1 (WANG BEIBEI [US] ET AL) 26 January 2023 (2023-01-26) <br> * paragraphs [0217] - [0221], [0232] - [0238], [0241], [0248] - [0251] * <br> * paragraphs [0263], [0269], [0316], [0317] * | 1-15 | |
| A | US 2021/059562 A1 (WU FANG-MING [TW]) 4 March 2021 (2021-03-04) <br> * abstract; figure 9A * <br> * paragraphs [0029] - [0034], [0041] * | 1-15 | |
| A | US 2013/030257 A1 (NAKATA ROBERT [US] ET AL) 31 January 2013 (2013-01-31) <br> * figure 6C * <br> * paragraphs [0006], [0076], [0096] - [0097] * | 1-15 | **TECHNICAL FIELDS SEARCHED (IPC)** <br><br> G01S <br> A61B |

The present search report has been drawn up for all claims

| Place of search | Date of completion of the search | Examiner |
|---|---|---|
| Munich | 9 April 2025 | Rodríguez González |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                                               

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 21 4533

This annex lists the patent family members relating to the patent documents cited in the above-mentioned European search report.
The members are as contained in the European Patent Office EDP file on
The European Patent Office is in no way liable for these particulars which are merely given for the purpose of information.

09-04-2025

| Patent document cited in search report | Publication date | Patent family member(s) | Publication date |
|---|---|---|---|
| US 2022373646 A1 | 24-11-2022 | CN 117177708 A | 05-12-2023 |
| | | EP 4284238 A1 | 06-12-2023 |
| | | US 2022373646 A1 | 24-11-2022 |
| | | WO 2022245189 A1 | 24-11-2022 |
| US 2023021342 A1 | 26-01-2023 | NONE | |
| US 2021059562 A1 | 04-03-2021 | CN 112438710 A | 05-03-2021 |
| | | TW 202108072 A | 01-03-2021 |
| | | US 2021059562 A1 | 04-03-2021 |
| US 2013030257 A1 | 31-01-2013 | AU 2012308234 A1 | 01-05-2014 |
| | | EP 2755720 A1 | 23-07-2014 |
| | | EP 4278962 A2 | 22-11-2023 |
| | | US 2013030257 A1 | 31-01-2013 |
| | | WO 2013040511 A1 | 21-03-2013 |

EPO FORM P0459

For more details about this annex : see Official Journal of the European Patent Office, No. 12/82

**REFERENCES CITED IN THE DESCRIPTION**

**Patent documents cited in the description**

- US 63604658